# EUROPÄISCHE PATENTSCHRIFT

(11) **EP 1 201 179 B1**
(45) Veröffentlichungstag und Bekanntmachung des Hinweises auf die Patenterteilung: **07.08.2002**
(21) Anmeldenummer: 00123081.2
(22) Anmeldetag: 24.10.2000
(51) Int. Cl.: A61B 1/07, A61B 1/00

(54) **Weisslichtquelle mit Leuchtdioden (LED) für Endoskop**
White-light source using LEDs for an endoscope
Source de lumière blanche utilisant des diodes électroluminescentes (DEL) pour endoscope

(43) Veröffentlichungstag der Anmeldung: 02.05.2002
(73) Patentinhaber: Karl Storz GmbH & Co. KG, 78532 Tuttlingen (DE)
(72) Erfinder: Irion, Klaus M., 78576 Liptingen (DE)
(74) Vertreter: Heuckeroth, Volker, Dr.

(56) Entgegenhaltungen:
- EP-A- 0 557 709
- WO-A-00/54655
- WO-A-98/02085
- DE-A- 19 715 510
- DE-U- 9 402 678
- DE-U- 29 922 755
- US-A- 5 241 170

## Beschreibung

Die Erfindung betrifft ein Endoskop, mit einem Schaft, einem im Schaft angeordneten Bildgebungssystem, und mit einem Beleuchtungssystem, das von einer Lichtquelle emittiertes Licht am distalen Ende des Schafts austreten läßt, wobei die Lichtquelle zumindest eine Leuchtdiode und zumindest einen Lichtleiter aufweist.

Ein derartiges Endoskop ist aus dem deutschen Gebrauchsmuster 299 22 755 bekannt.

Endoskope werden sowohl auf technischem Gebiet als auch auf medizinischem Gebiet verwendet.

Anwendungsbeispiele für ein technisches Endoskop sind die Inspektion von schwer zugänglichen und daher mit dem bloßen Auge nicht beobachtbaren Hohlräumen in Maschinen, Motoren, Turbinen, Reaktionsräumen usw.

In der medizinischen Endoskopie werden Endoskope in der minimal-invasiven Chirurgie ggf. in Kombination mit chirurgischen Instrumenten zu Untersuchungszwecken oder für Operationen unter Sichtkontrolle oder auch zur Applikation von diagnostischem oder therapeutischem Licht verwendet.

Ein erster Bestandteil derartiger Endoskope ist das Bildgebungssystem. Das Bildgebungssystem dient zum Empfangen von Beobachtungslicht aus dem Beobachtungsraum bzw. Operationsraum und zur Übertragung von Bildinformationen von distal nach proximal.

Das Bildgebungssystem kann herkömmlich in einem optischen Bildübertragungssystem bestehen, das ein Objektiv im distalen Ende des Schafts, ein sich daran proximal anschließendes Linsensystem, beispielsweise in Form von Stablinsen, oder ein geordnetes Faserbündel und ein proximales Okular aufweist, durch das mit dem Auge beobachtet werden kann, oder an das eine Kamera anschließbar ist.

Das Bildgebungssystem kann aber auch im distalen Ende des Schafts ein Kameramodul mit einer Abbildungsoptik und einem Bildaufnehmer-Chip aufweisen, beispielsweise in Form eines CCD-Chips, der die Lichtsignale in elektrische Signale umwandelt, die über elektrische Leitungen nach proximal übertragen werden und außerhalb des Endoskops auf einer Bildwiedergabeeinheit als reelles Bild zur visuellen Darstellung gebracht werden.

Ein zweiter Bestandteil derartiger Endoskope, auf den sich die vorliegende Erfindung bezieht, ist das Beleuchtungssystem. Das Beleuchtungssystem dient zur Übertragung von Licht von proximal nach distal, um den Beobachtungsraum bzw. Untersuchungsraum mit Licht auszuleuchten. Für eine helle Ausleuchtung wird üblicherweise Weißlicht verwendet, während für andere Anwendungen, beispielsweise für Untersuchungen mit Anregungslicht, auch farbiges Licht verwendet wird.

Bei derzeitigen angewandten Endoskopen weist das Beleuchtungssystem eine externe Lichtquelle auf, üblicherweise auf der Basis einer Xenon- oder Halogenlampe. Das Endoskop ist dann über ein Lichtleitkabel mit der externen Lichtquelle verbunden.

Abgesehen davon, daß derartige Xenon- oder Halogen-Lichtquellen sehr kostenaufwendige Apparaturen sind, besteht ein weiterer Nachteil derartiger herkömmlicher Endoskope darin, daß die Verbindung des Endoskops über ein Lichtleitkabel mit der Lichtquelle bei der Anwendung des Endoskops störend ist. Aufgrund des langen Übertragungswegs von der Lichtquelle zur distalen Spitze des Endoskops, der 2 bis 3 m betragen kann, treten außerdem Verluste in der Lichtstärke des aus dem distalen Ende des Endoskops austretenden Lichts auf, die durch eine entsprechend leuchtstarke Lichtquelle mit entsprechendem Kostenaufwand ausgeglichen werden müssen.

Bei dem aus dem oben genannten Gebrauchsmuster bekannten Endoskop wird auf eine externe Lichtquelle verzichtet, indem in das Endoskop zumindest eine Leuchtdiode (LED) integriert ist. Da Leuchtdioden inzwischen mit großer Lichtstärke verfügbar sind, kann der Beobachtungs- bzw. Operationsraum mit einer Leuchtdiode als Lichtquelle ausreichend hell ausgeleuchtet werden. Eine externe kostenaufwendige Lichtquelle kann dadurch eingespart werden. Leuchtdioden haben darüber hinaus den Vorteil, daß im Falle der Applikation von farbigem Licht die Farbqualität von Licht einer Leuchtdiode besser ist, als wenn eine Weißlichtquelle mit einem Farbfilter verwendet wird. Darüber hinaus wird das bei Anwendungen des Endoskops störende Lichtleitkabel zwischen der externen Lichtquelle und dem Endoskop überflüssig, wodurch einerseits weitere Kosten eingespart und andererseits die Handhabung des Endoskops verbessert wird.

Leuchtdioden wären grundsätzlich aufgrund ihrer Bauform und ihres Wirkungsgrades geeignet für den Einsatz in der Endoskopie, sowohl für eine kontinuierliche als auch für eine gepulste Beleuchtung. Nachteilig an Leuchtdioden ist jedoch, daß die Leistungsdichte des von Lichtdioden emittierten Lichts nicht sehr hoch ist und daß leistungsstärkere Leuchtdioden relativ schmalbandig abstrahlen. So existieren beispielsweise leistungsstärkere Dioden, die vor allem im roten und infraroten Bereich schmalbandig abstrahlen. Demgegenüber bestehen Anforderungen in der Endoskopie, daß stets naturgetreue Farbbilder und keine "Einfarb-" (beispielsweise Rot-) bzw. Schwarz-Weißbilder benötigt werden.

Aus der WO-A-00 54655 ist ein ophthalmologisches Beleuchtungssystem bekannt, das Licht emittiert, das bezüglich seiner Farbe variabel einstellbar ist. Das Licht wird durch drei Leuchtdioden mit unterschiedlichen Emissionswellenlängen erzeugt, wobei das Licht der einzelnen Leuchtdioden in einem Mischer zu weißem Licht oder Licht mit einer bestimmten eingestellten Farbe gemischt wird.

Der Erfindung liegt die Aufgabe zugrunde, ein Endoskop der eingangs genannten Art dahingehend weiterzubilden, daß möglichst naturgetreue Farbbilder durch das Endoskop beobachtet werden können, wobei die Beleuchtungseinrichtung möglichst platzsparend ausgebildet sein soll.

Erfindungsgemäß wird diese Aufgabe dadurch gelöst, daß die Lichtquelle zumindest zwei Leuchtdioden aufweist, die Licht in unterschiedlichen Spektralbereichen emittieren, und daß das Licht der zumindest zwei Leuchtdioden spektral additiv gemischt aus dem Beleuchtungssystem austritt, wobei Einkoppelmittel zum Einkoppeln des von den zumindest zwei Leuchtdioden emittierten Lichts in den zumindest einen Lichtleiter vorgesehen sind und die Einkoppelmittel als Strahlteiler mit zumindest teilweise reflektierenden Schichten ausgebildet sind, wobei jeder Leuchtdiode ein Strahlteiler zugeordnet ist, und die Strahlteiler in axialer Verlängerung des Lichtleiters hintereinander angeordnet sind, wobei jeder Strahlteiler im Spektralbereich des von der zugeordneten Leuchtdiode emittierten Lichts reflektierend und in dem oder den Spektralbereich(en) des von den nachgeordneten Leuchtdioden emittierten Lichts durchlässig ist.

Durch die vorgesehene additive Farbmischung des Lichts zumindest zweier Leuchtdioden wird vorteilhafterweise erreicht, daß ein möglichst farbgetreues endoskopisches Bild wiedergegeben wird, weil es aufgrund dieser Ausgestaltung der Erfindung möglich ist, das Licht zweier spektral-schmalbandiger Leuchtdioden durch eine additive Farbmischung zu Weißlicht zu mischen. Wird der Untersuchungs- bzw. Beobachtungsraum mit Weißlicht ausgeleuchtet, kann ein farbgetreues endoskopisches Bild erhalten werden. Diese Maßnahme ermöglicht es insbesondere, leistungsstarke Leuchtdioden zu verwenden, wobei deren naturgemäße spektrale Schmalbandigkeit ("Einfarbigkeit") durch die additive Mischung des von den Leuchtdioden emittierten Lichts zur Erzielung eines farbgetreuen endoskopischen Bildes ausgeglichen werden kann. Des weiteren wird vorteilhafterweise der Effekt ausgenutzt, daß sich die Lichtstärke der zumindest zwei Leuchtdioden zu einer hohen Lichtstärke addiert.

Das Beleuchtungssystem weist zumindest einen Lichtleiter auf, und es sind Einkoppelmittel zum Einkoppeln des von den zumindest zwei Leuchtdioden, vorzugsweise von den mehreren Leuchtdioden emittierten Lichts in den Lichtleiter vorgesehen. Die Einkoppelmittel stellen eine vorteilhafte Maßnahme dar, um Licht insbesondere von mehreren Leuchtdioden in ein und denselben Lichtleiter einzukoppeln und dadurch die Möglichkeit einer additiven Farbmischung des von den einzelnen Leuchtdioden emittierten Lichts zu erreichen.

Die Einkoppelmittel sind dabei als Strahlteiler mit zumindest teilweise reflektierenden Schichten ausgebildet, wobei jeder Leuchtdiode ein Strahlteiler zugeordnet ist.

Diese Maßnahme stellt eine vorteilhafte Möglichkeit dar, im Falle einer Anordnung von mehreren Leuchtdioden das von diesen emittierte Licht in ein und denselben Lichtleitereingangsquerschnitt einzukoppeln, ohne daß die Leuchtdioden notwendigerweise in geradliniger Verlängerung des Lichtleiters angeordnet werden müssen, sondern auch platzsparend seitlich vom Lichtleiter angeordnet sein können.

Die Strahlteiler sind in axialer Verlängerung des Lichtstrahls hintereinander angeordnet sind, und jeder Strahlteiler ist im Spektralbereich des von der zugeordneten Leuchtdiode emittierten Lichts reflektierend und in dem oder den Spektralbereich(en) des von den nachgeordneten Leuchtdioden emittierten Lichts durchlässig.

Durch die Hintereinanderanordnung der Strahlteiler in axialer Verlängerung des Lichtleiters kann das von den LED's emittierte Licht vorteilhafterweise parallel zur Lichtleiterachse eingekoppelt werden, wodurch Reflexionsverluste bei der Lichtausbreitung im Lichtleiter gering gehalten werden. Durch die Ausgestaltung der Strahlteiler, die in bestimmten Spektralbereichen durchlässig und in bestimmten Spektralbereichen reflektierend sind, wird der Vorteil erreicht, daß mehrere Strahlteiler/Leuchtdioden-Anordnungen, beispielsweise drei Strahlteiler/Leuchtdioden-Anordnungen für eine blaue, grüne und rote Leuchtdiode hintereinander geschaltet werden können. Durch die Hintereinanderschaltung mehrerer teilweise durchlässiger Strahlteiler wird der weitere Vorteil erreicht, daß der Lichtleiterquerschnitt selbst sehr gering gehalten werden kann, was in der Endoskopie erwünscht ist.

Dabei ist es insbesondere bevorzugt, wenn die zumindest zwei Leuchtdioden Licht in zueinander spektral komplementären Spektralbereichen emittieren.

Durch diese Maßnahme wird aufgrund der additiven Farbmischung des von den zumindest zwei Leuchtdioden emittierten Lichts exakt Weißlicht hoher Lichtstärke erzeugt. Beispielsweise kann eine Leuchtdiode mit einer maximalen Abstrahlleistung im Frequenzbereich der Spektralfarbe Blau zusammen mit einer Leuchtdiode mit einer maximalen Abstrahlleistung im Spektralbereich der Farbe Orange verwendet werden, oder es kann beispielsweise eine im roten Spektralbereich abstrahlende Leuchtdiode zusammen mit einer im grünen Spektralbereich abstrahlenden Leuchtdiode verwendet werden.

In einer weiteren bevorzugten Ausgestaltung weist die Lichtquelle zumindest drei Leuchtdioden auf, die Licht vorzugsweise im blauen, grünen und roten Spektralbereich emittieren.

Eine derartige Leuchtdiodenanordnung mit zumindest drei Leuchtdioden im blauen, grünen und roten Spektralbereich eröffnet vorteilhafterweise nicht nur die Möglichkeit der Erzeugung von noch intensitätsstärkerem Weißlicht, sondern eröffnet auch die Möglichkeit einer Farb- und/oder Intensitätsmodulation bei entsprechender gezielter Ansteuerung der drei einzelnen Leuchtdioden und entsprechender Mischung des von den einzelnen Leuchtdioden emittierten Lichts.

In einer weiteren bevorzugten Ausgestaltung ist zumindest eine Leuchtdiode eine Leuchtdiode mit einer spektralschmalbandigen Abstrahlcharakteristik hoher Lichtstärke, insbesondere eine High-Brightness- oder eine Ultrahigh-Brightness-Leuchtdiode.

Solche heute verfügbaren HB- bzw. UHB-LED's, die üblicherweise auf der Basis von Halbleiterverbindungen wie GaN, ZnSe oder SiC hergestellt werden, besitzen vorteilhafterweise eine sehr hohe Lichtstärke und hervorragende Farbqualität mit einer spektralschmalbandigen Abstrahlcharakteristik. Die schmalbandige Abstrahlcharakteristik der einzelnen Leuchtdioden kann für bestimmte Anwendungen vorteilhaft sein, bei denen mit farbigem Licht hoher Farbqualität gearbeitet werden sollte, oder beispielsweise bei einem therapeutischen Einsatz von Licht in der photodynamischen Therapie. In Verbindung mit der additiven Farbmischung des Lichts verschiedener LED's haben solche LED's den Vorteil, daß Weißlicht mit hoher Lichtstärke erzeugt werden kann.

Dabei ist es bevorzugt, wenn die zumindest zwei Leuchtdioden und die Einkoppelmittel am proximalen Ende des Lichtleiters angeordnet sind.

Die Anordnung der zumindest einen Leuchtdiode und der Einkoppelmittel am proximalen Ende des Lichtleiters und somit am proximalen Ende des Endoskops hat den Vorteil, daß am proximalen Ende des Endoskops mehr Bauraum zur Verfügung steht als am distalen Ende, so daß das erfindungsgemäße Endoskop im Bereich seines distalen Endes sehr schmalbauend ausgeführt werden kann, was insbesondere für endoskopische Anwendungen im medizinischen Bereich im Rahmen der minimal-invasiven Chirurgie gefordert wird.

In einer weiteren bevorzugten Ausgestaltung entspricht der Querschnitt jedes Strahlteilers dem aktiven Querschnitt des Lichtleiters.

Diese Ausgestaltung ist insbesondere für endoskopische Anwendungen von Vorteil, da sie es ermöglicht, Licht hoher Leistungsdichte in einen geringen Lichtleiterquerschnitt einzukoppeln und dabei den gesamten Lichtleiterquerschnitt optimal zur Lichtübertragung zu nutzen.

In einer weiteren bevorzugten Ausgestaltung ist eine Mehrzahl von Anordnungen aus jeweils mehreren Leuchtdioden und Strahlteilern vorgesehen, und ist jeder dieser Anordnungen ein weiterer Lichtleiter zugeordnet, und sind die weiteren Lichtleiter an einen gemeinsamen Lichtleiter angekoppelt, in dem das von den Leuchtdioden emittierte Licht nach distal geführt wird.

Durch diese Ausgestaltung wird die Flexibilität des Endoskops hinsichtlich der Farbvariation und/oder der Intensitätsänderung des Lichts weiter erhöht, außerdem kann die Lichtstärke und damit die Helligkeit der Ausleuchtung des Beobachtungs- bzw. Untersuchungsraums noch weiter verbessert werden.

In einer weiteren bevorzugten Ausgestaltung sind die zumindest zwei Leuchtdioden und/oder die Einkoppelmittel im Endoskop selbst angeordnet.

Hierbei ist von Vorteil, daß das so ausgestaltete Endoskop mit der integrierten Lichtquelle, bestehend aus den zumindest zwei Leuchtdioden und gegebenenfalls den Einkoppelmitteln, als Ganzes eine autarke Einheit bildet, die unabhängig von externen Lichtquellen ist.

In einer weiteren bevorzugten Ausgestaltung sind die zumindest zwei Leuchtdioden und /oder die Einkoppelmittel vom Endoskop abnehmbar.

Diese Maßnahme hat den Vorteil, daß die eine bzw. die mehreren Leuchtdioden gegen andere bereit gehaltene Leuchtdioden ausgetauscht werden können. Die Einkoppelmittel in Form der zuvor erwähnten Strahlteiler können fest in das Endoskop integriert sein. Jedoch können auch die Einkoppelmittel vorteilhafterweise vom Endoskop abnehmbar ausgestaltet sein.

In einer weiteren bevorzugten Ausgestaltung sind die zumindest zwei Leuchtdioden gepulst oder kontinuierlich ansteuerbar.

Die gepulste Ansteuerung der Leuchtdioden ist insbesondere zur Erzeugung von Stroboskopeffekten, beispielsweise für die Stroboskopie zur Untersuchung der Stimmbandfunktion vorteilhaft. Die gepulste Ansteuerung kann auch in Kombination mit einer endoskopischen Kamera erfolgen, so daß pro Strahlteiler-Leuchtdiodenanordnung mehrere Pulse von Dioden unterschiedlicher spektraler Zusammensetzung einsetzbar sind und so eine Farb- und Intensitätsmodulation erzielt werden kann. Auf diese Weise besteht eine Optimierungsmöglichkeit hinsichtlich der Farbtreue und/oder der Intensität.

Dabei ist es bevorzugt, wenn Leuchtdioden mit unterschiedlichen Abstrahlspektren unterschiedlich ansteuerbar sind.

Auf diese Weise läßt sich vorteilhafterweise die zuvor erwähnte Farbmodulation erreichen.

In einer weiteren bevorzugten Ausgestaltung ist eine Ansteuerungseinrichtung für die zumindest zwei Leuchtdioden im Endoskop angeordnet.

Durch diese Maßnahme ist das Endoskop vorteilhafterweise eine autonome Baueinheit und benötigt keine externe Ansteuerungsvorrichtung mit störenden Verbindungskabeln zum Ansteuern der zumindest zwei Leuchtdioden.

Ebenso ist es bevorzugt, wenn eine Stromversorgung für die zumindest zwei Leuchtdioden im Endoskop angeordnet ist.

Auf diese Weise wird das erfindungsgemäße Endoskop insgesamt autonom von externen Versorgungen. Beispielsweise können die Leuchtdioden über eine Batterie oder Akkuversorgung betrieben werden, die beispielsweise im Handstück des Endoskops angeordnet ist.

Dabei ist es bevorzugt, wenn die Stromversorgung zusammen mit den zumindest zwei Leuchtdioden als Einheit vom Endoskop abnehmbar ist.

Hierbei ist von Vorteil, daß die Einheit aus Stromversorgung und den zumindest zwei Leuchtdioden, gegebenenfalls mit den Einkoppelmitteln, auch unabhängig von dem Endoskop und autark als Lichtquelle verwendet werden kann.

In einer weiteren bevorzugten Ausgestaltung sind die zumindest zwei Leuchtdioden und/oder die Einkoppelmittel in einer vom Endoskop abnehmbaren Kupplungseinheit zum licht-und bildübertragenden Verbinden des Endoskops mit einem Kameramodul angeordnet.

Auch diese Maßnahme stellt eine vorteilhafte Ausgestaltung des Endoskops dar, bei der die zumindest zwei Leuchtdioden und/oder die Einkoppelmittel nicht unmittelbar im Endoskop selbst angeordnet sind, sondern in einer vom Endoskop abnehmbaren Kupplungseinheit, die zum licht- und bildübertragenden Verbinden des Endoskops mit einem Kameramodul dient. Eine solche Kupplungseinheit ist in der DE 197 15 510 A1 beschrieben. Im Unterschied zu der dort beschriebenen Kupplungseinheit kann bei dem erfindungsgemäßen Endoskop nach Integration der zumindest zwei Leuchtdioden in die Kupplungseinheit das am proximalen Ende der Kupplungseinheit vorgesehene Lichtleitkabel zur Verbindung mit einer externen Lichtquelle wegfallen, da eine externe Lichtquelle nun nicht mehr benötigt wird.

Wenn, wie in einer weiteren bevorzugten Ausgestaltung angegeben, auch noch die Stromversorgung für die zumindest zwei Leuchtdioden in der Kupplungseinheit angeordnet ist, ist die Kupplungseinheit hinsichtlich der Lichterzeugung und Lichtemission vollkommen autark. Als Stromversorgung können in der Kupplungseinheit wiederaufladbare Batterien aufgenommen sein, die über ein entsprechendes Ladegerät, das vorzugsweise unmittelbar über Kontakte an die Kupplungseinheit anschließbar ist, aufgeladen werden können.

Eine noch größere Autonomität der Kupplungseinheit und damit des Endoskops wird durch eine weitere bevorzugte Ausgestaltung erreicht, nach der in der Kupplungseinheit eine Sendereinheit zur drahtlosen Übertragung eines von dem Kameramodul aufgenommenen Videobildes angeordnet ist.

Während bei der in der DE 197 15 510 A1 beschriebenen Kupplungseinheit für die Bildübertragung am proximalen Ende der Kupplungseinheit ein entsprechendes elektrisches Kabel vorgesehen ist, kann auch dieses Kabel durch die vorstehend beschriebene Ausgestaltung nunmehr wegfallen, wodurch zusammen mit der zuvor beschriebenen Ausgestaltung der autonomen Stromversorgung die Kupplungseinheit vollständig kabellos ausgeführt werden kann und somit die Handhabung des Endoskops mit der Kupplungseinheit nicht durch Kabel behindert wird. Zumindest kann jedoch durch die vorliegende Erfindung auch ohne die zuvor beschriebene Sendeeinheit das in der Regel stärker störende Lichtleitkabel zum Anschließen der Kupplungseinheit an eine Lichtquelle eingespart werden.

Weitere Merkmale und Vorteile ergeben sich aus der nachfolgenden Beschreibung der beigefügten Zeichnung.

Ausführungsbeispiele der Erfindung sind in der Zeichnung dargestellt und werden mit Bezug auf diese hier noch näher beschrieben. Es zeigen:
- Fig. 1: eine schematische Darstellung eines Endoskops in einer Längsschnittdarstellung;
- Fig. 2: eine schematische Darstellung eines Ausschnitts des Beleuchtungssystems des Endoskops in Fig. 1;
- Fig. 3: einen Ausschnitt eines Beleuchtungssystems für das Endoskop in Fig. 1 gemäß einem weiteren Ausführungsbeispiel;
- Fig. 4: eine schematische Darstellung der Intensitätsverteilung der einzelnen Leuchtdioden des Endoskops in Fig. 1 und des Reflexions- und Transmissionsverhaltens von Strahlteilern zur Einkopplung des von den Leuchtdioden emittierten Lichts in den Lichtleiter des Endoskops in Fig. 1; und
- Fig. 5: eine ausschnittsweise Darstellung eines Endoskops mit einer Kupplungseinheit zum licht- und bildübertragenden Verbinden eines Endoskops mit einem Kameramodul.

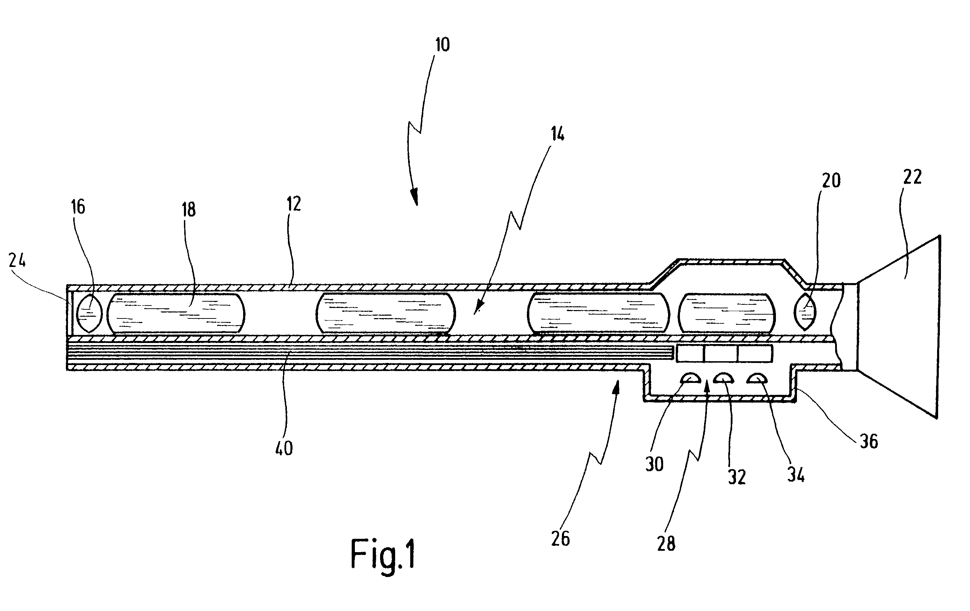

In Fig. 1 ist ein mit dem allgemeinen Bezugszeichen 10 versehenes Endoskop schematisch dargestellt.

Das Endoskop 10 ist ein medizinisches Endoskop, wobei die Erfindung jedoch nicht auf medizinische Endoskope beschränkt ist, sondern auch in technischen Endoskopen Anwendung finden kann.

Das Endoskop 10 weist einen Schaft 12 auf.

In dem Schaft 12 ist ein Bildgebungssystem 14 angeordnet, das ein Objektiv 16 im Bereich des distalen Endes des Schafts 12, ein Linsensystem 18 in Form einer Mehrzahl von Stablinsen und eine Okularoptik 20 umfaßt. Das Bildgebungssystem 14 kann jedoch auch ein geordnetes Faserbündel oder ein Kameramodul mit einem optoelektronischen Bildaufnehmer am distalen Ende des Schafts 12 aufweisen.

Am proximalen Ende des Schafts 12 ist eine Okularmuschel 22 angeordnet.

Am distalen Ende des Schafts 12 ist ein Deckglas 24 angeordnet.

Das Endoskop weist weiterhin ein Beleuchtungssystem 26 auf. Das Beleuchtungssystem 26 umfaßt eine Lichtquelle 28, die bei dem Endoskop 10 in Form von drei Leuchtdioden, einer ersten Leuchtdiode 30, einer zweiten Leuchtdiode 32 und einer dritten Leuchtdiode 34 ausgebildet ist.

Die Leuchtdioden 30, 32 und 34 sind am proximalen Ende des Endoskops 10 angeordnet, und zwar innerhalb eines am Schaft 12 ausgebildeten Endoskopgehäuses 36.

Die Leuchtdioden 30, 32 und 34 sind bevorzugt sogenannte High-Brightness- oder Ultrahigh-Brightness-Leuchtdioden (HB-LED's bzw. UHB-LED's), vorzugsweise auf der Basis einer Materialverbindung mit Halbleitereigenschaften.

Eine solche Materialverbindung mit Halbleitereigenschaften ist GaN.

Die Leuchtdioden 30, 32 und 34 können jedoch auch Leuchtdioden auf der Basis von Silizium sein.

Des weiteren eignen sich als Leuchtdioden 30, 32 und 34 auch auf einem organischen Halbleitermaterial basierende Leuchtdioden (OLED), beispielsweise auf der Basis von Poly-Phenylen-Vinyl (PPV). Aber auch niedermolekulare Verbindungen wie Aluminiumchinolat (Alq) oder Metall-Chelat-Komplexe sind Basismaterialien für OLED's.

Eine weitere Alternative zu den zuvor genannten Leuchtdiodenmaterialien sind Karbon-Nano-Tube-Strukturen, die sich im Rahmen der neueren Forschung ebenfalls als für lichtstarke Lichtquellen geeignet erwiesen haben.

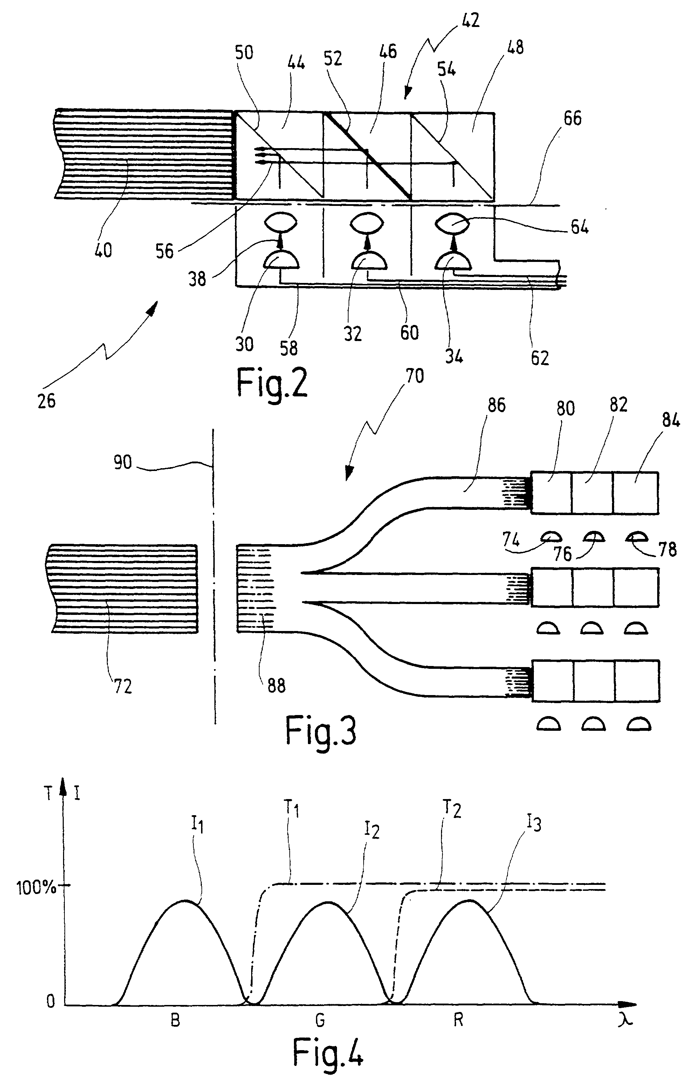

Die Leuchtdioden 30, 32, 34 sind insbesondere spektral schmalbandig emittierende Leuchtdioden, deren spektrale Lichtemissionscharakteristiken in Fig. 4 dargestellt sind. In Fig. 4 ist in einem Diagramm die Intensitätsverteilung I in Abhängigkeit von der Wellenlänge λ des emittierten Lichts aufgetragen.

Mit I₁ ist die spektrale Verteilung der Intensität des von der ersten Leuchtdiode 30 emittierten Lichts dargestellt. Die erste Leuchtdiode 30 weist eine maximale Abstrahlleistung im Blau-Bereich (B) des Spektrums auf. Mit I₂ ist die Intensitätskurve der zweiten Leuchtdiode 32 dargestellt, deren maximale Abstrahlleistung im Grün-Bereich (G) liegt. Mit I₃ ist der spektrale Intensitätsverlauf der dritten Leuchtdiode 34 dargestellt, deren maximale Abstrahlleistung im Rot-Bereich (R) des Spektrums liegt.

In Fig. 2 ist die Leuchtdiodenanordnung aus den Leuchtdioden 30, 32 und 34 vergrößert dargestellt.

Die Leuchtdioden 30, 32 und 34 sind in Längsrichtung des Endoskops 10 hintereinander angeordnet. Die Leuchtdioden 30, 32 und 34 sind dabei so orientiert, daß ihre Hauptabstrahlrichtung quer zur Längsrichtung des Endoskops 10 verläuft, wie mit Pfeilen 38 angedeutet ist.

Gemäß Fig. 1 und 2 weist das Beleuchtungssystem 26 des Endoskops 10 ferner einen Lichtleiter 40 auf, der aus einem Glasfaserbündel gebildet wird. Der Lichtleiter 40 erstreckt sich vom distalen Ende des Schafts 12 bis etwa auf Höhe der ersten Leuchtdiode 30.

Zum Einkoppeln des von den Leuchtdioden 30, 32 und 34 emittierten Lichts in den Lichtleiter 40 sind Einkoppelmittel 42 vorgesehen. Die Einkoppelmittel 42 werden durch Strahlteiler gebildet, wobei der ersten Leuchtdiode 30 ein erster Strahlteiler 44, der zweiten Leuchtdiode 32 ein zweiter Strahlteiler 46 und der dritten Leuchtdiode 34 ein dritter Strahlteiler 48 zugeordnet ist. Jeder der Strahlteiler 44, 46 und 48 weist eine zumindest teilweise reflektierende Schicht auf, und zwar weist der erste Strahlteiler 44 eine zumindest teilweise reflektierende Schicht 50, der zweite Strahlteiler 46 eine zumindest teilweise reflektierende Schicht 52 und der dritte Strahlteiler 48 eine zumindest teilweise reflektierende Schicht 54 auf.

Die Reflexions- und Transmissionseigenschaften in Abhängigkeit von der Lichtwellenlänge der zumindest teilweise reflektierenden Schichten 50 und 52 der Strahlteiler 44 und 46 sind in Fig. 4 ebenfalls dargestellt.

Dazu ist in Fig. 4 die Transmissivität T in Abhängigkeit von der Wellenlänge λ aufgetragen.

Mit T₁ ist die Transmissionskurve der zumindest teilweise reflektierenden Schicht 50 des ersten Strahlteilers 44 dargestellt. Wie aus Fig. 4 hervorgeht, ist die Transmissivität T₁ der zumindest teilweise reflektierenden Schicht 50 des ersten Strahlteilers 44 im Blau-Bereich 0, d.h. Licht im Blau-Bereich wird von der zumindest teilweise reflektierenden Schicht 50 vollständig reflektiert. Dies bedeutet, daß das von der ersten Leuchtdiode 30 im Blau-Bereich emittierte Licht von dem Strahlteiler 44 in das proximale Ende des Lichtleiters 40 eingekoppelt wird.

Hingegen ist die Transmissivität T₁ der zumindest teilweise reflektierenden Schicht 50 des ersten Strahlteilers 44 im Grün- und Rot-Bereich 100 %, d.h. Licht im Grün- und dem Rot-Bereich wird von der zumindest teilweise reflektierenden Schicht 50 vollständig hindurchgelassen.

Die zumindest teilweise reflektierende Schicht 52 des zweiten Strahlteilers 46 ist gemäß der Transmissionskurve T₂ im Blauund Grün-Bereich 0, d.h. von der zweiten Leuchtdiode 32 im Grün-Bereich emittiertes Licht wird von dem Strahlteiler 46 zu 100 % reflektiert. Da der erste Strahlteiler 44 im Grün-Bereich vollständig durchlässig ist, wird das von der zweiten Leuchtdiode 32 emittierte Licht im Grün-Bereich ebenfalls in das proximale Ende des Lichtleiters 40 eingekoppelt.

Die zumindest teilweise reflektierende Schicht 52 des zweiten Strahlteilers 46 ist hingegen für Licht im Rot-Bereich zu 100 % durchlässig.

Die zumindest teilweise reflektierende Schicht 54 des dritten Strahlteilers 48 ist für Licht im Rot-Bereich zu 100 % reflektierend. Das von der dritten Leuchtdiode 34 im Rot-Bereich emittierte Licht wird somit von dem dritten Strahlteiler 48 reflektiert und aufgrund der Transmissivität des ersten und zweiten Strahlteilers 44 und 46 im Rot-Bereich ebenfalls in das proximale Ende des Lichtleiters 40 eingekoppelt.

Mit Pfeilen 56 ist die Einkoppelrichtung für das jeweilige von der ersten Leuchtdiode 30, der zweiten Leuchtdiode 32 und der dritten Leuchtdiode 34 emittierte Licht angedeutet.

Aufgrund der vorstehend beschriebenen Art der Einkopplung des von den Leuchtdioden 30, 32 und 34 emittierten Lichts in den Lichtleiter 40 findet eine additive Mischung des von den Leuchtdioden 30, 32 und 34 emittierten Lichts statt, so daß aus dem Beleuchtungssystem 26, d.h. aus dem Lichtleiter 40 am distalen Ende des Schafts 12 des Endoskops 10 entsprechend additiv gemischtes Licht austritt, wenn Licht von zumindest zwei der Leuchtdioden 30, 32, 34 in den Lichtleiter 40 eingekoppelt wird. Im Falle der vorstehend beschriebenen Leuchtdiodenanordnung mit der blau leuchtenden Leuchtdiode 30, der grün leuchtende Leuchtdiode 32 und der rot leuchtenden Leuchtdiode 34 kann somit bei entsprechender Mischung Weißlicht hoher Intensität erzeugt werden.

Weißlicht kann jedoch anstelle einer Leuchtdiodenanordnung mit wie vor beschrieben drei Leuchtdioden auch bereits mit nur zwei Leuchtdioden erreicht werden, wenn die eine Leuchtdiode in einem Spektralbereich Licht emittiert, und die andere Leuchtdiode in einem dazu komplementären Spektralbereich Licht emittiert.

Mit der zuvor beschriebenen Leuchtdiodenanordnung mit drei Leuchtdioden im blauen, grünen und roten Frequenzspektrum können bei entsprechender Ansteuerung der einzelnen Leuchtdioden 30, 32 und 34 Farbmodulationen und/oder Intensitätsmodulationen durchgeführt werden, um so eine Optimierung des Lichts, das am distalen Ende aus dem Lichtleiter 40 austritt, hinsichtlich der Farbtreue oder der Intensität des Lichts, zu erzielen.

Die Leuchtdioden 30, 32, 34 können gepulst oder kontinuierlich angesteuert werden. Dabei können die Leuchtdioden 30, 32 und 34 auch unterschiedlich angesteuert werden, um beispielsweise die Farb- und/oder Intensitätsmodulation vorzunehmen, wodurch neben Weißlicht auch farbiges Licht einer gewünschten Farbe erzeugt werden kann.

Für die Leuchtdioden 30, 32 und 34 sind schematisch elektrische Leitungen 58, 60 und 62 dargestellt, die mit einer entsprechenden Ansteuerungseinrichtung und einer Stromversorgung verbunden sind. Die Ansteuerungseinrichtung und die Stromversorgung, die in den Figuren nicht dargestellt sind, sind bevorzugt ebenfalls in das Endoskop 10 integriert, so daß das Endoskop 10 autonom betrieben werden kann.

Die Strahlteiler 44, 46 und 48 sind hinsichtlich ihres Querschnittes dem aktiven Querschnitt des Lichtleiters 40 angepaßt, so daß eine optimale, den gesamten aktiven Querschnitt des Lichtleiters 40 erfassende Einkopplung des von den Leuchtdioden 30, 32 und 34 emittierten Lichts erfolgt.

Des weiteren ist für jede der Leuchtdioden 30, 32 und 34 jeweils ein Element 64 zur Strahlfokussierung zugeordnet.

Die Anordnung aus den Leuchtdioden 30, 32 und 34 ist des weiteren von der Anordnung aus den Strahlteilern 44, 46 und 48 abnehmbar, wie in Fig. 2 mit einer Linie 66 angedeutet ist. Die Leuchtdioden 30, 32 und 34 können somit gegen andere Leuchtdioden nach Bedarf ausgetauscht werden.

In Fig. 3 ist noch ein weiteres Ausführungsbeispiel eines Beleuchtungssystems 70 dargestellt, das einen dem Lichtleiter 40 vergleichbaren Lichtleiter 72 aufweist. Das Beleuchtungssystem 70 weist eine Mehrfachanordnung aus jeweils drei Leuchtdioden 74, 76 und 78 sowie drei Strahlteilern 80, 82 und 84 auf, wobei die Leuchtdioden 74, 76 und 78 und die Strahlteiler 80, 82 und 84 entsprechend den Leuchtdioden 30, 32, 34 und den Strahlteilern 44, 46 und 48 ausgebildet sind.

Insgesamt sind drei derartiger Anordnungen aus den Leuchtdioden 74, 76 und 78 und den Strahlteilern 80, 82 und 84 bei dem Beleuchtungssystem 70 vorgesehen.

Jeder der Anordnungen aus den Leuchtdioden 74, 76 und 78 und den Strahlteilern 80, 82 und 84 ist ein weiterer Lichtleiter 86 zugeordnet, in den das von den Leuchtdioden 74, 76 und 78 emittierte Licht additiv eingekoppelt wird. Die drei weiteren Lichtleiter 86 laufen dann zu einem gemeinsamen Lichtleiterende 88 zusammen, von dem aus dann das Licht in den Lichtleiter 72 eingekoppelt wird.

Das Lichtleiterende 88 mit den Leuchtdioden/Strahlteileranordnungen ist von dem Lichtleiter 72 abkoppelbar, wie mit einer Linie 90 angedeutet ist.

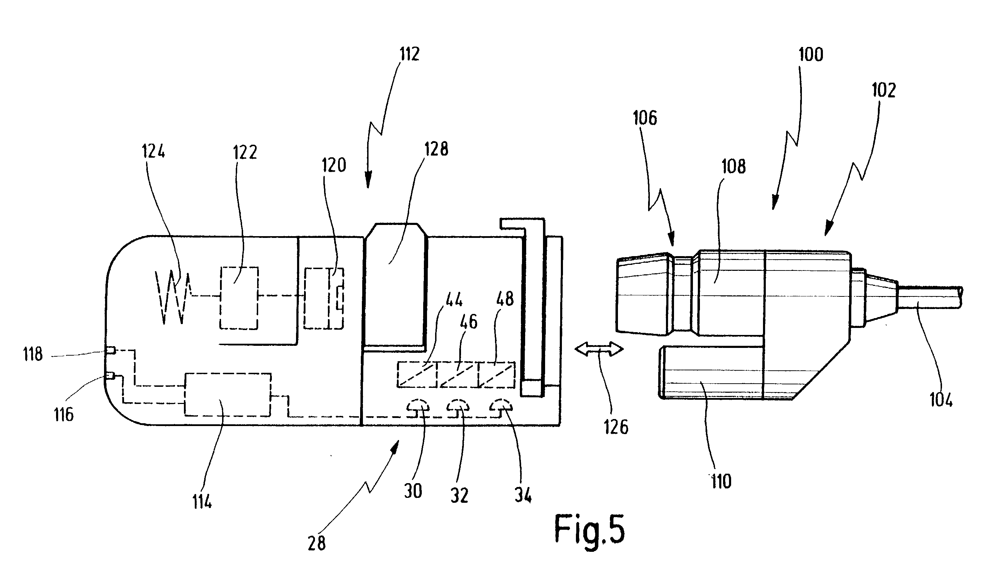

Schließlich ist in Fig. 5 ein weiteres Ausführungsbeispiel eines Endoskops 100 dargestellt, das insgesamt aus zwei Teilen zusammensetzbar ist. Ein erstes Teil 102 umfaßt einen Endoskopschaft 104, an dessen proximalem Ende eine Steckkupplung 106 angeordnet ist. Die Steckkupplung 106 weist einen ersten Zapfen 108, in dem das proximalseitige Ende des Bildgebungssystems aufgenommen ist, und einen zweiten Zapfen 110 auf, in dem das proximale Ende des Beleuchtungssystems, das einen Lichtleiter umfaßt, angeordnet ist.

Die wiederum mit dem Bezugszeichen 28 versehene Lichtquelle 28, die beispielsweise die bereits genannten Leuchtdioden 30, 32, 34 umfaßt, ist nunmehr in einer von dem Endoskop 100 abnehmbaren Kupplungseinheit 112 angeordnet.

Die Kupplungseinheit 112 entspricht hinsichtlich der Ausführung des Kupplungsmechanismus der in der DE 197 15 510 A1 beschriebenen Kupplungseinheit, so daß diesbezüglich auf die dortige Beschreibung verwiesen wird.

Die gegenüber der dort beschriebenen Kupplungseinheit abgewandelten Merkmale sind in Fig. 5 mit unterbrochenen Linien eingezeichnet, wozu auch die Leuchtdioden 30, 32 und 34 gehören. Wie mit Bezug auf die vorherigen Ausführungsbeispiele beschrieben, sind jeder Leuchtdiode 30, 32 und 34 wiederum Einkoppelmittel in Form der Strahlteiler 44, 46 und 48 zugeordnet. Als Stromversorgung für die Leuchtdioden 30, 32 und 34 ist in der Kupplungseinheit 112 beispielsweise eine Batterie oder eine wiederaufladbare Batterie 114 angeordnet. Im Fall, daß eine wiederaufladbare Batterie vorgesehen ist, sind ferner vorzugsweise am proximalen Ende des Gehäuses der Kupplungseinheit 112 Steckerkontakte 116 und 118 vorgesehen, über die die Kupplungseinheit 112 an ein entsprechendes Batterieladegerät angeschlossen werden kann, um die wiederaufladbare Batterie 114 zu laden.

Die Kupplungseinheit 112 umfaßt ferner ein Kameramodul 120 mit einem entsprechenden Bildaufnehmerchip, der das vom Bildübertragungssystem des Endoskops 100 über den Zapfen 108 in die Kupplungseinheit 112 übertragene Licht in entsprechend elektrische Signale umwandelt. Das Kameramodul 120 ist weiterhin mit einem Sender 122 verbunden, über den die vom Kameramodul 120 erzeugten Signale über eine entsprechende Antenne 124 drahtlos abgestrahlt werden können, die dann von einem entsprechenden Empfänger empfangen werden und als reale Bilder auf einem Videobildschirm zur Darstellung gebracht werden.

Die Kupplungseinheit 112 und das Endoskop 100 werden im Gebrauch gemäß einem Doppelpfeil 126 zusammengesteckt, wie dies in der DE 197 15 510 A1 beschrieben ist. Im zusammengesteckten Zustand liegen die Strahlteiler 44, 46 und 48 auf Höhe des Zapfens 110 sowie das Kameramodul 120 auf Höhe des Zapfens 108.

Dem Kameramodul 120 ist entsprechend noch eine Fokussiereinheit 128 vorgeschaltet.

Gegenüber der in der DE 197 15 510 A1 beschriebenen Kupplungseinheit benötigt die Kupplungseinheit 112 weder einen Anschluß für ein externes Lichtleitkabel noch einen Anschluß für ein elektrisches Kabel zum Übertragen der Signale vom Kameramodul 120 auf eine Videoeinheit. Vielmehr ist die Kupplungseinheit 112 vollkommen autark und frei von störenden Verbindungskabeln.

## Patentansprüche

1. Endoskop (10, 100), mit einem Schaft (12, 104), einem im Schaft (12, 104) angeordneten Bildgebungssystem (14), und mit einem Beleuchtungssystem (26; 70), das von einer Lichtquelle (28) emittiertes Licht am distalen Ende des Schafts (12) austreten läßt, wobei die Lichtquelle (28) zumindest zwei Leuchtdioden (30, 32, 34; 74, 76, 78), die Licht in unterschiedlichen Spektralbereichen emittieren, und zumindest einen Lichtleiter (40; 72; 86) aufweist, **dadurch gekennzeichnet, daß** das Licht der zumindest zwei Leuchtdioden (30, 32, 34; 74, 76, 78) spektral additiv gemischt aus dem Beleuchtungssystem (26; 70) austritt, wobei Einkoppelmittel (42) zum Einkoppeln des von den zumindest zwei Leuchtdioden (30, 32, 34; 74, 76, 78) emittierten Lichts in den zumindest einen Lichtleiter (40; 72; 86) vorgesehen sind, und die Einkoppelmittel als Strahlteiler (44, 46, 48; 80, 82, 84) mit zumindest teilweise reflektierenden Schichten (50, 52, 54) ausgebildet sind, wobei jeder Leuchtdiode (30, 32, 34; 74, 76, 78) ein Strahlteiler zugeordnet ist, und die Strahlteiler (44, 46, 48; 80, 82, 84) in axialer Verlängerung des zumindest einen Lichtleiters (40, 72) hintereinander angeordnet sind, wobei jeder Strahlteiler (44, 46, 48; 80, 82, 84) im Spektralbereich des von der zugeordneten Leuchtdiode (30, 32, 34; 74, 76, 78) emittierten Lichts reflektierend und in dem oder den Spektralbereich(en) des von den nachgeordneten Leuchtdioden (32, 34; 76, 78) emittierten Lichts durchlässig ist.

2. Endoskop nach Anspruch 1, **dadurch gekennzeichnet, daß** die zumindest zwei Leuchtdioden (30, 32, 34; 74, 76, 78) Licht in zueinander spektral komplementären Spektralbereichen emittieren.

3. Endoskop nach Anspruch 1 oder 2, **dadurch gekennzeichnet, daß** die Lichtquelle (28) zumindest drei Leuchtdioden (30, 32, 34; 74, 76, 78) aufweist, die Licht vorzugsweise im blauen, grünen und roten Spektralbereich emittieren.

4. Endoskop nach einem der Ansprüche 1 bis 3, **dadurch gekennzeichnet, daß** zumindest eine der Leuchtdioden (30, 32, 34; 74, 76, 78) eine spektral schmalbandige Abstrahlcharakteristik und eine hohe Lichtstärke aufweist, insbesondere eine High-Brightness oder eine Ultrahigh-Brightness-Leuchtdiode ist.

5. Endoskop nach einem der Ansprüche 1 bis 4, **dadurch gekennzeichnet, daß** die zumindest zwei Leuchtdioden (30, 32, 34; 74, 76, 78) und die Einkoppelmittel (42) am proximalen Ende des Lichtleiters (40; 72; 86) angeordnet sind.

6. Endoskop nach einem der Ansprüche 1 bis 5, **dadurch gekennzeichnet, daß** der Querschnitt jedes Strahlteilers (44, 46, 48; 80, 82, 84) dem aktiven Querschnitt des Lichtleiters (40; 86) entspricht.

7. Endoskop nach einem der Ansprüche 1 bis 6, **dadurch gekennzeichnet, daß** eine Mehrzahl von Anordnungen aus mehreren Leuchtdioden (74, 76, 78) und Strahlteilern (80, 82, 84) vorgesehen ist, und daß jeder dieser Anordnungen ein Lichtleiter (86) zugeordnet ist, und daß die Lichtleiter (86) an einen gemeinsamen Lichtleiter (72) angekoppelt sind, in dem das von den Leuchtdioden (74, 76, 78) emittierte Licht nach distal geführt wird.

8. Endoskop nach einem der Ansprüche 1 bis 7, **dadurch gekennzeichnet, daß** die zumindest zwei Leuchtdioden (30, 32, 34; 74, 76, 78) und/oder gegebenenfalls die Einkoppelmittel (42) im Endoskop (10, 100) selbst angeordnet sind.

9. Endoskop nach Anspruch 8, **dadurch gekennzeichnet, daß** die zumindest zwei Leuchtdioden (30, 32, 34; 74, 76, 78) und/oder die Einkoppelmittel (42) vom Endoskop (10, 100) abnehmbar sind.

10. Endoskop nach einem der Ansprüche 1 bis 9, **dadurch gekennzeichnet, daß** die zumindest zwei Leuchtdioden (30, 32, 34; 74, 76, 78) gepulst oder kontinuierlich ansteuerbar sind.

11. Endoskop nach Anspruch 10, **dadurch gekennzeichnet, daß** Leuchtdioden mit unterschiedlichen Abstrahlspektren unterschiedlich ansteuerbar sind.

12. Endoskop nach Anspruch 10 oder 11, **dadurch gekennzeichnet, daß** eine Ansteuerungseinrichtung für die zumindest zwei Leuchtdioden (30, 32, 34; 74, 76, 78) im Endoskop angeordnet ist.

13. Endoskop nach einem der Ansprüche 1 bis 12, **dadurch gekennzeichnet, daß** eine Stromversorgung für die zumindest zwei Leuchtdioden (30, 32, 34; 74, 76, 78) im Endoskop (10) angeordnet ist.

14. Endoskop nach Anspruch 13, **dadurch gekennzeichnet, daß** die Stromversorgung zusammen mit den zumindest zwei Leuchtdioden (30, 32, 34; 74, 76, 78) als Einheit vom Endoskop abnehmbar ist.

15. Endoskop nach einem der Ansprüche 1 bis 7, 10 oder 11, **dadurch gekennzeichnet, daß** die zumindest zwei Leuchtdioden (30, 32, 34) und/oder die Einkoppelmittel (42) in einer vom Endoskop (100) abnehmbaren Kupplungseinheit (112) zum licht- und bildübertragenden Verbinden des Endoskops (100) mit einem Kameramodul (120) angeordnet sind.

16. Endoskop nach Anspruch 15, **dadurch gekennzeichnet, daß** in der Kupplungseinheit (112) ein Sender (122) zur drahtlosen Übertragung eines von dem Kameramodul (120) aufgenommenen Videobildes angeordnet ist.

17. Endoskop nach Anspruch 15 oder Anspruch 16, **dadurch gekennzeichnet, daß** in der Kupplungseinheit (112) eine Stromversorgung für die zumindest zwei Leuchtdioden (30, 32, 34) angeordnet ist.

## Claims

1. An endoscope (10, 100), comprising a shaft (12, 104), an imaging system (14) arranged in the shaft (12, 104), and an illuminating system (26; 70) which allows light emitted by a light source (28) to emerge at the distal end of the shaft (12), wherein the light source (28) comprises at least two light emitting diodes (30, 32, 34; 74, 76, 78) which emit light in different spectral ranges and at least one optical waveguide (40; 72; 86), **characterized in that** the light of the at least two light emitting diodes (30, 32, 34; 74, 76, 78) emerges spectrally additively mixed from the illuminating system (26; 70), wherein light coupling means (42) are provided for coupling in the light emitted by the at least two light emitting diodes (30, 32, 34; 74, 76, 78) into the at least one optical waveguide (40; 72; 86), and the light coupling means are configured as beam splitters (44, 46, 48; 80, 82, 84) which comprise at least partly reflecting layers (50, 52, 54), wherein a beam splitter is assigned to each light emitting diode (30, 32, 34; 74, 76, 78), and the beam splitters (44, 46, 48; 80, 82, 84) are arranged one behind the other in axial extension of the at least one optical waveguide (40; 72), wherein each beam splitter (44, 46, 48; 80, 82, 84) is reflecting in the spectral range of the light emitted by the assigned light emitting diode (30, 32, 34; 74, 76, 78) and permeable in the spectral range(s) of the light emitted by the light emitting diode(s) (32, 34; 76, 78) arranged therebehind.

2. The endoscope of claim 1, **characterized in that** the at least two light emitting diodes (30, 32, 34; 74, 76, 78) emit light in spectral ranges that are spectrally complementary to each other.

3. The endoscope of claim 1 or 2, **characterized in that** the light source (28) comprises at least three light emitting diodes (30, 32, 34; 74, 76, 78) which emit light preferably in the blue, green, and red spectral range.

4. The endoscope of anyone of claims 1 through 3, **characterized in that** the at least two light emitting diodes (30, 32, 34; 74, 76, 78) comprise a spectrally narrow-band radiation characteristic and a high light intensity, in particular are high-brightness or ultrahigh-brightness light emitting diodes.

5. The endoscope of anyone of claims 1 through 4, **characterized in that** the at least two light emitting diodes (30, 32, 34; 74, 76, 78) and the light coupling means (42) are arranged at the proximal end of the optical waveguide (40; 72; 86).

6. The endoscope of anyone of claims 1 through 5, **characterized in that** the cross section of each beam splitter (44, 46, 48; 80, 82, 84) corresponds to the active cross section of the optical waveguide (40; 86).

7. The endoscope of anyone of claims 1 through 6, **characterized in that** a plurality of arrangements of several light emitting diodes (74, 76, 78) and beam splitters (80, 82, 84) is provided, and that to each of these arrangements is assigned an optical waveguide (86), and that the optical waveguides (86) are coupled to a common optical waveguide (72), in which the light emitted by the light emitting diodes (74, 76, 78) is guided distalwards.

8. The endoscope of anyone of claims 1 through 7, **characterized in that** the at least two light emitting diodes (30, 32, 34; 74, 76, 78) and/or, if necessary, the light coupling means (42) are arranged in the endoscope (10, 100).

9. The endoscope of claim 8, **characterized in that** the at least two light emitting diodes (30, 32, 34; 74, 76, 78) and/or, if necessary, the light coupling means (42) are detachable from the endoscope (10, 100).

10. The endoscope of anyone of claims 1 through 9, **characterized in that** the at least two light emitting diodes (30, 32, 34; 74, 76, 78) can be driven in pulsed or continuous manner.

11. The endoscope of claim 10, **characterized in that** light emitting diodes having different radiation spectrums can be driven differently.

12. The endoscope of claim 10 or 11, **characterized in that** a drive circuit for the at least two light emitting diodes (30, 32, 34; 74, 76, 78) is arranged in the endoscope.

13. The endoscope of anyone of claims 1 through 12, **characterized in that** a current supply for the at least two light emitting diodes (30, 32, 34; 74, 76, 78) is arranged in the endoscope (10).

14. The endoscope of claim 13, **characterized in that** the current supply is, together with the at least two light emitting diodes (30, 32, 34; 74, 76, 78), detachable as a unit from the endoscope.

15. The endoscope of anyone of claims 1 through 7, 10 or 11, **characterized in that** the at least two light emitting diodes (30, 32, 34) and/or, if necessary, the light coupling means (42) are arranged in a coupling unit (112) for light and image transmitting connection of the endoscope (100) with a camera module (120), which coupling unit (112) is detachable from the endoscope (100).

16. The endoscope of claim 15, **characterized in that** a sender (122) is arranged in the coupling unit (112) for wireless transmission of a video image picked up by the camera module (120).

17. The endoscope of claim 15 or claim 16, **characterized in that** a current supply is arranged for the at least two light emitting diodes (30, 32, 34) in the coupling unit (112).

## Revendications

1. Endoscope (10, 100) comprenant une tige (12, 104), un système émetteur d'images (14) disposé dans la tige (12, 104), et comprenant un système d'éclairage (26 ; 70) laissant s'échapper, à l'extrémité distale de la tige (12), de la lumière émise par une source lumineuse (28), la source lumineuse (28) présentant au moins deux diodes luminescentes (30, 32, 34 ; 74, 76, 78) qui émettent de la lumière dans des domaines spectraux différents, et au moins un guide de lumière (40 ; 72 ; 86), **caractérisé en ce que** la lumière des diodes luminescentes (30, 32, 34 ; 74, 76, 78), au nombre minimum de deux, sort du système d'éclairage (26 ; 70) en s'additionnant et en se mélangeant spectralement, des moyens d'injection (42) étant prévus pour injecter la lumière émise par les diodes luminescentes (30, 32, 34 ; 74, 76, 78), au nombre minimum de deux, dans le guide de lumière (40 ; 72 ; 86), au nombre minimum d'un, et les moyens d'injection étant conformés en séparateurs de faisceaux (44, 46, 48 ; 80, 82, 84) présentant des couches (50, 52, 54) au moins partiellement réfléchissantes, un séparateur de faisceaux étant associé à chaque diode luminescente (30, 32, 34 ; 74, 76, 78), et les séparateurs de faisceaux (44, 46, 48 ; 80, 82, 84) étant disposés les uns derrière les autres, dans le prolongement axial du guide de lumière (40, 72), au nombre minimum d'un, chaque séparateur de faisceaux (44, 46, 48 ; 80, 82, 84) étant réflecteur, dans le domaine spectral de la lumière émise par la diode luminescente associée (30, 32, 34 ; 74, 76, 78) et étant transparent, dans le ou les domaine(s) spectral/spectraux de la lumière émise par les diodes luminescentes associées (32, 34 ; 76, 78).

2. Endoscope selon la revendication 1, **caractérisé en ce que** les diodes luminescentes (30, 32, 34 ; 74, 76, 78), au nombre minimum de deux, émettent de la lumière dans des domaines spectraux complémentaires.

3. Endoscope selon la revendication 1 ou 2, **caractérisé en ce que** la source lumineuse (28) comprend au moins trois diodes luminescentes (30, 32, 34 ; 74, 76, 78), qui émettent de la lumière, de préférence, dans le domaine spectral du bleu, du vert et du rouge.

4. Endoscope selon l'une des revendications 1 à 3, **caractérisé en ce qu'**au moins une des diodes luminescentes (30, 32, 34 ; 74, 76, 78) présente une caractéristique de rayonnement spectral à bande étroite et une intensité lumineuse élevée, et est, en particulier, une diode luminescente à haute luminosité ou une diode luminescente à ultra-haute luminosité.

5. Endoscope selon l'une des revendications 1 à 4, **caractérisé en ce que** les diodes luminescentes (30, 32, 34 ; 74, 76, 78), au nombre minimum de deux, et les moyens d'injection (42) sont placés à l'extrémité proximale du guide de lumière (40 ; 72 ; 86).

6. Endoscope selon l'une des revendications 1 à 5, **caractérisé en ce que** la section transversale de chaque séparateur de faisceaux (44, 46, 48 ; 80, 82, 84) correspond à la section transversale active du guide de lumière (40 ; 86).

7. Endoscope selon l'une des revendications 1 à 6, **caractérisé en ce qu'**est prévue une majorité d'agencements constitués de plusieurs diodes luminescentes (74, 76, 78) et de séparateurs de faisceaux (80, 82, 84), et **en ce qu'**est associé, à chacun de ces agencements, un guide de lumière (86), et **en ce que** les guides de lumière (86) sont couplés à un guide de lumière (72) commun, grâce au fait que la lumière émise par les diodes luminescentes (74, 76, 78) est guidée en direction de l'extrémité distale.

8. Endoscope selon l'une des revendications 1 à 7, **caractérisé en ce que** les diodes luminescentes (30, 32, 34 ; 74, 76, 78), au nombre minimum de deux, et/ou, le cas échéant, les moyens d'injection (42) sont disposés dans l'endoscope (10, 100) proprement dit.

9. Endoscope selon la revendication 8, **caractérisé en ce que** les diodes luminescentes (30, 32, 34 ; 74, 76, 78), au nombre minimum de deux, et/ou les moyens d'injection (42) peuvent être retirés de l'endoscope (10, 100).

10. Endoscope selon l'une des revendications 1 à 9, **caractérisé en ce que** les diodes luminescentes (30, 32, 34 ; 74, 76, 78), au nombre minimum de deux, sont susceptibles d'être commandées par impulsions ou en continu.

11. Endoscope selon la revendication 10, **caractérisé en ce que** des diodes luminescentes présentant des spectres de rayonnement différents sont susceptibles d'être commandées différemment.

12. Endoscope selon la revendication 10 ou 11, **caractérisé en ce qu'**un dispositif de commande destiné aux diodes luminescentes (30, 32, 34 ; 74, 76, 78), au nombre minimum de deux, est monté dans l'endoscope.

13. Endoscope selon l'une des revendications 1 à 12, **caractérisé en ce qu'**une alimentation en courant destiné aux diodes luminescentes (30, 32, 34 ; 74, 76, 78), au nombre minimum de deux, est prévue dans l'endoscope (10).

14. Endoscope selon la revendication 13, **caractérisé en ce que** l'alimentation en courant, qui forme une unité avec les diodes luminescentes, (30, 32, 34 ; 74, 76, 78), au nombre minimum de deux, peut être retirée de l'endoscope.

15. Endoscope selon l'une des revendications 1 à 7, 10 ou 11, **caractérisé en ce que** les diodes luminescentes (30, 32, 34), au nombre minimum de deux, et/ou les moyens d'injection (42) sont montés dans une unité de couplage (112) susceptible d'être retirée de l'endoscope (100), afin que l'endoscope (100) puisse être relié, avec transmission de lumière et d'images, à un module formant caméra (120).

16. Endoscope selon la revendication 15, **caractérisé en ce qu'**est disposé, dans l'unité de couplage (112), un émetteur (122) pour la transmission sans fil d'une image vidéo prise par le module formant caméra (120).

17. Endoscope selon la revendication 15 ou la revendication 16, **caractérisé en ce qu'**est placée, dans l'unité de couplage (112), une alimentation en courant destinée aux diodes luminescentes (30, 32, 34), au nombre minimum de deux.
